Europäisches Patentamt

European Patent Office

Office européen des brevets

(19)

(11) **EP 1 006 197 A1**

(12) **EUROPEAN PATENT APPLICATION**

(43) Date of publication:
**07.06.2000 Bulletin 2000/23**

(51) Int. Cl.$^7$: **C12N 15/87**, A61K 48/00

(21) Application number: **98403057.7**

(22) Date of filing: **04.12.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant: **TRANSGENE S.A.**
**67082 Strasbourg Cédex (FR)**

(72) Inventor: **Jacobs, Eric**
**67140 Stotzheim (FR)**

(74) Representative:
**VOSSIUS & PARTNER**
**Siebertstrasse 4**
**81675 München (DE)**

(54) **Use of an immuno complex for the preparation of a therapeutic composition useful for transfecting a polynucleotide into macropinocyte cells**

(57)    Described is the use of an immuno complex, which comprises at least one antibody or a reactive part thereof and at least one polynucleotide, for the preparation of a therapeutic composition for the introduction of a polynucleotide into a macropinocyte target cell wherein said immuno complex has a particle size selected between 0.5 µm and 6 µm.

**Description**

The present invention relates to the use of an immuno complex for the preparation of a therapeutic composition for targeting transfection of a polynucleotide into a macropinocyte cell. Such a composition is useful in gene therapy, vaccination, and any therapeutic or prophylactic situation in which a gene-based product is administered to such a cell *in vitro, ex vivo or in vivo*.

Gene therapy has generally been conceived as principally applicable to heritable deficiency diseases (cystic fibrosis, dystrophies, haemophilias, etc.) where permanent cure may be effected by introducing a functional gene. However, a much larger group of diseases, notably acquired diseases (cancer, AIDS, multiple sclerosis, etc.) might be treatable by transiently engineering host cells to produce beneficial proteins. Many genes involved in said deficiencies or of therapeutic interest have been identified. Direct expression of these genes within patients should contribute to a significant amelioration of the symptoms by expression of the functional polypeptide in targeted tissues. A specific application of gene therapy is vaccination. In this regard, the immunogenic product encoded by the polynucleotide introduced in cells of a vertebrate may be produced and/or secreted, and be processed and presented by Antigen Presenting Cells (APC) cells in the context of the major histocompatibility antigens, thereby eliciting an immuno response against the expressed immunogen. The Antigen Presenting Cells (APC) such as macrophages and dendritic cells, especially "sentinel cells", play essential roles in the initiation of the immuno response. They are first competent for antigen capture and intracellular antigen processing and second they express MHC class I and/or II genes which encode transmembrane glycoproteins involved in presentation of said processed antigenic peptides to $CD8^+$ and/or $CD4^+$ T-cells, and also produce specific accessory protein, thereby leading to T-cell activation (Debrick et al., J. Immunol. 147 (1991), 2846; Reis et al., J. Exp. Med. 178 (1993), 509; Kovacsovics-Bankowski et al., PNAS 90 (1993), 4942; Kovacsovics-Bankowski et al., Science 267 (1995), 243; Svensson et al., J. Immunol. 158 (1997), 4229; Norbury et al., Eur. J. Immunol. 27 (1997), 280). For vaccinal purposes, it can therefore be advantageous to make available a gene therapy system which can direct gene transfer into such APC cells, said gene encoding an antigenic polypeptide which can, after its intracellular production, be processed and presented to $CD8^+$ and/or $CD4^+$ cells by MHC class I and MHC class II complex, respectively, on the surface of said cells.

Success of gene therapy depends on the efficient delivery to and expression of genetic information within the cells of a living organism. In this respect, a variety of techniques, resulting in either transient expression of the gene of interest, referred to as transient transfection, or permanent transformation of the host cells resulting from incorporation of the polynucleotide into the host genome, have been proposed in order to introduce functional polynucleotides into cells. Most delivery mechanisms used to date involve viral vectors, especially adeno- and retroviral vectors. Viruses have developed diverse and highly sophisticated mechanisms to achieve this goal including crossing of the cellular membrane, escape from lysosomal degradation, delivery of their genome to the nucleus and, consequently, have been used in many gene delivery applications in vaccination or gene therapy applied to humans. The use of viruses suffers from a number of disadvantages: retroviral vectors cannot accommodate large-sized DNA (for example, the dystrophin gene which is around 13 Kb), the retroviral genome is integrated into host cell DNA and may thus cause genetic changes in the recipient cell and infectious viral particles could disseminate in the organism or in the environment and adenoviral vectors can induce a strong immuno response in treated patients (Mc Coy et al., Human Gene Therapy 6 (1995), 1553-1560; Yang et al., Immunity 1 (1996), 433-442). Nevertheless, despite these drawbacks, viral vectors are currently the most useful delivery systems because of their efficiency.

In 1990, Wolff et al. (Science 247, 1465-1468) have shown that injection of naked RNA or DNA, without any special delivery system, directly into mouse skeletal muscle results in expression of reporter genes within the muscle cells. Nevertheless, although these results indicate that nucleic acid by itself is capable of cellular uptake and is expressed in certain cells *in vivo*, the efficiency of the transfection actually observed remains very limited due, in particular, to the polyanionic nature of nucleic acids which limits their passage through negatively-charged cell membranes.

Various methods have been proposed in the literature based on the use of non-viral synthetic vectors to improve intracellular uptake of nucleic acids which present potential advantages with respect to large-scale production, safety, targeting of transfectable cells, low immunogenicity, and the capacity to deliver large fragments of DNA. Thus, in 1989, Felgner et al. (Nature 337, 387-388) proposed the use of cationic lipids in order to facilitate the introduction of large anionic molecules such as nucleic acids into cells. These cationic lipids are capable of forming complexes with anionic molecules, thus tending to neutralize the negative charges of these molecules allowing to compact the complex, and favoring its introduction into the cell. Examples for lipid-mediated transfection compounds are DOTMA (Felgner et al., PNAS 84 (1987), 7413-7417), DOGS or Transfectam™ (Behr et al., PNAS 86 (1989), 6982-6986), DMRIE or DORIE (Felgner et al., Methods 5 (1993), 67-75), DC-CHOL (Gao et Huang, BBRC 179 (1991), 280-285), DOTAP™ (McLachlan et al., Gene Therapy 2 (1995), 674-622) or Lipofectamine™.

Other non-viral delivery systems have been developed which are based on polymer-mediated transfection.

There have been many reports on the use for cellular delivery of anionic polymers such as, for example, polyamidoamine (Haensler et Szoka, Bioconjugate Chem. 4 (1993), 372-379), dendritic polymer (WO 95/24221), polyethylene imine or polypropylene imine (WO 96/02655), polylysine (US-A-5,595,897 or FR-A-2 719 316).

Moreover, WO 97/02840 describes a product coupling a nucleic acid and an immunovector using p-benzoquinone as a linker, characterized in that said immunovector is capable of enabling the nucleic acid to be internalised into cells and in that said immunovector has an affinity for the cellular DNA to such an extent that it can transfer the coupled nucleic acid into or to the immediate vicinity of the cell nucleus. Earlier approaches in order to find pathogenic lupus autoantibodies have shown that such anti-DNA antibodies can cross both the cell and nuclear membranes to localize within nucleus in living cells of multiple organs after administration to normal mice. Nevertheless, in 1998, Avrameas et al. (PNAS 95, 5601-5606) have indicated that cellular uptake and subsequent nuclear localization of these immunoglobulins were dependent on their antigen binding region and that only polyreactive anti-DNA antibodies were able to penetrate into the living cells tested, without cell specificity. However, very little is known concerning the mechanisms which enable the interaction of the complexes, formed between the polynucleotide and the proposed vectors, with the cell membranes and the transfer of these complexes into the cell and the nucleus. Ongoing researches remain highly empirical and do not provide any satisfactory model for expression of a particular gene into a particular cell. In addition, the vast majority of transfected cells is not APC cells. Proposed solutions require to first identify a targeting molecule which will specifically target the gene uptake.

Several proteins or polypeptides have been proposed as targeting molecules for delivery of macromolecules into cells. Receptor-mediated gene transfer makes use of the ability of receptors on the surface of a variety of differenciated cells to efficiently bind and internalize a ligand and permits the targeting of DNA uptake into specific tissues. Said systems include DNA of interest, a protein containing the receptor-targeting ligand, and in most cases a linking polycation. Hereinafter are examples of receptor/targeting ligand systems: asialoglycoprotein / asialooromucoid-poly(L-lysine), transferrin/transferrin-poly(L-lysine), insulin/albulin-insulin conjugate, and particularly mannose/mannosylated poly(L-lysine) for targeting the macrophages (for a review, Perales et al., European J. Bioch. 226 (1994), 255-266). However, to be useful in gene transfer, it is critical that both the chemical properties and physical interactions of the reagents involved in the design of the DNA delivery vehicle be rigourously characterized. Furthermore, one of the major disavantages of this targeting system is that DNA/ligand complexes are difficult to prepare and there is no system which provides a solution for direct transfection of gene of interest into all APCs.

Thus, the technical problem underlying the present invention is to provide means and methods for a targeted delivery of nucleic acid molecules into specific cells, in particular into macropinocyte cells.

This problem has been solved by the embodiments as characterized in the claims.

Thus, the present invention relates to the use of an immuno complex which comprises at least one antibody or a reactive part thereof and at least one polynucleotide, for the preparation of a therapeutic composition for the introduction of a polynucleotide into a macropinocyte target cell wherein said immuno complex has a particle size selected between 0.5 μm and 6 μm, and preferably has a particle size of at least 1 μm.

It has been surprisingly found that the formation of immuno complexes containing antibodies and a polynucleotide provides an efficient system to achieve targeted cellular delivery of a nucleic acid into vertebrate macropinocyte cells which can therefore find an application in in vivo gene therapy, and preferably in in vivo vaccination. The described system for delivering nucleic acid molecules is in particular advantageous because it allows to specifically direct nucleic acid sequences into, e.g., macrophages and dendritic cells, which are also known as "sentinel" APCs involved in active immunoprotection of mammals against pathogenic agents. Thus, the possibility to specifically transfect these cells whith nucleic acid molecules is of importance for vaccination, since it is possible to express antigens in these cells. These are then further processed intracellularly and presented to T-cells by MHC glycoproteins leading to T-cell activation and a specific immune response directed against a pathogenic agent or cell expressing said antigenic peptide. Possible applications are, e.g., anti-tumoral vaccination, anti-viral vaccination etc.

The size of the immuno complex of the use according to the invention may be selected for optimal use in a particular application. Measurements of the complex size can be achieved by a number of techniques including, but not limited to, dynamic laser light scattering (photon correlation spectroscopy, PCS), electronic microscopy, freeze fracture electronic microscopy as well as other techniques known to those skilled in the art (see, Washington, Particle Size Analysis in Pharmaceutics and other Industries, Ellis Horwood, New York (1992), 135-169). Antigen Presenting Cells (APC), specially "sentinel" APCs, possess different mechanisms of antigen uptake: (a) capture of antigens by surface receptors such as receptors for immunoglobulins (Fc) or for complement, available on granulocytes, monocytes or macrophages, allows efficient delivery of the antigen to the processing compartment after receptor-mediated phagocytosis; and (b)antigens that fail to bind to cell surface receptors can still be taken up by fluid phase pinocytosis. Fluid phase uptake can occur via distinct mechanism: micropinocytosis, i.e. uptake of small vesicles (~0.1 μm) via clathrin-coated pits and macropinocytosis, i.e. uptake of larger vesicles (with a size varying between about 0.5 μm and about 6μm) mediated by membrane ruffling (Sallusto et al., J. Exp. Med. 182 (1995), 389-400) which does not require any attachement to the cell membrane. While micropinocytosis occurs constitutively in many cells, macropinocytosis is limited to a few cell types, more particularly to APCs such as macrophages, dendritic cells and epithelial cells stimulated by growth factors (Racoosin et al., J. Cell Sci. 102 (1992), 867-880). Therefore, according to the present invention, the term "macropinocyte cells" refers to cells which are capable of macropinocytosis events as previously defined, and to take up macromolecules, preferably ranging in size from about 0.5 μm to about 6 μm, into the cytoplasm. Preferably they are selected from the group consisting of macrophages and dendritic cells.

The term "immuno complex" in the scope of the present invention means a complex formed between at least one antibody and at least one antigen. The antibody involved in said immuno complex is specific for said antigen and therefore is able to recognize and to bind to its specific antigen.

The antibody included in the immuno complex can specifically recognize and bind to the polynucleotide present in said immuno complex or to at least one antigenic compound formulated with said polynucleotide. Furthermore, the antibody may only recognize the polynucleotide and not said antigenic compound formulated with said polynucleotide or, vice versa, only the antigenic compound formulated with the polynucleotide and not the polynucleotide (monoreactive antibody). In another embodiment the antibody is able to recognize and bind to said polynucleotide and to said antigenic compound formulated with the polynucleotide (polyreactive antibody).

Examples for the antigenic compound with which the polynucleotide may be formulated are polypeptides, preferably viral polypeptides, cationic lipids and/or cationic polymers, eventually substituted with an immunogenic element. Candidate compunds are widely described in the literature related to synthetic vectors for gene therapy, the immunogenic (or antigenic) properties of which can be easily tested, for example, by direct administration to mammals. These antigenic compounds preferably are selected among compounds comprising at least one peptide, one sugar part or any other suitable immunogenic part.

In a preferred embodiment the antibody is an anti-polynucleotide antibody, more preferably an anti-DNA antibody. "Anti-polynucleotide antibody" or "anti-DNA antibody" designates an antibody which is able to recognize and to bind with a single stranded or/and double stranded polynucleotide or a DNA molecule, respectively. According to the invention, said antibody can be a polyclonal, or preferably, a monoclonal antibody. In a preferred embodiment said antibody is an autoantibody obtained from serum of a mammal with an autoimmuno disease (see for example Yanase et al., J. Clin. Invest. 100 (1997), 25-31). Such an anti-polynucleotide antibody can be obtained, e.g., by immunization of mammals with all or part of a polynucleotide as previously described in Marion et al. (Methods 11 (1997), 3-11) and by production in hybridoma cells according to methods wellknown in the art (see for example Avraemas et al., PNAS 95 (1998), 5601-5606). Moreover, anti-polynucleotide antibodies and more specifically anti-DNA antibodies are already commercially available (Interchim). The term "antibody" encompasses whole immunoglobulins of any class(mono- and multimeric), preferably IgG or IgM, chimeric antibodies and hybrid antibodies with dual or multiple epitope specificities, and "reactive parts thereof" which means antibody fragments derived from said antibodies which can be selected from the group consisting of $F(ab')_2$, $F(ab)_2$ Fab', Fab, Fv, sFv and minimal recognition units, including hybrid fragments and anti-idiotypes (US 4,699,880) which pertain the ability to recognize and to bind specifically to polynucleotides or to DNA.

The term "polynucleotide" as used in the scope of the present invention means a DNA and/or RNA fragment, single or double-stranded, linear or circular, natural or synthetic, modified or not (see US 5525711, US 4711955, US 5792608 or EP 302 175 for modification examples) defining a fragment or a portion of a nucleic acid, without size limitation. It may be, *inter alia*, a genomic DNA, a cDNA, an mRNA, an antisense RNA, a ribozyme, or DNA encoding such RNAs. "Polynucleotides" and "nucleic acids" are synonyms with regard to the present invention. The polynucleotide may be in the form of a linear polynucleotide, and preferably in the form of a plasmid. The polynucleotide can also be an oligonucleotide which is to be delivered to the cell, e.g., for antisense or ribozyme functions. According to the invention, the polynucleotide is preferably a naked polynucleotide (Wolff et al., Science 247 (1990), 1465-1468) or is preferably formulated with at least one compound, preferably an antigenic compound, such as polypeptides, preferably viral polypeptides, or cationic lipids, or cationic polymers which can participate in the uptake of the polypeptide into the cells (see Ledley, Human Gene Therapy 6 (1995), 1129-1144 for a review), each of which could also be considered as an antigenic compound. Preferably, the polynucleotide contains at least one coding sequence that can be transcribed and translated to generate a polypeptide of interest. The genetic information necessary for expression by a target cell comprises all the elements required for transcription of DNA into RNA and, if necessary, for translation of mRNA into a polypeptide. Transcriptional promoters suitable for use in various vertebrate systems are well known. For example, suitable promoters include viral promoters like RSV, MPSV, SV40, CMV or 7.5k, vaccinia promoter, inducible promoters, etc. The polynucleotide can also include intron sequences, targeting sequences, transport sequences, sequences involved in replication or integration. Said sequences have been reported in the literature and can be readily obtained by those skilled in the art. The polynucleotide can also be modified in order to be stabilized with specific components as spermine. According to the invention, the polynucleotide can be homologous or heterologous to the target cells into which it is introduced. Examples of polypeptides encoded by the polynucleotide are enzymes, hormones, cytokines, membrane receptors, structural polypeptides, transport polypeptides, tumoral, viral or infectious antigens, adhesines, ligands, transcription factors, translation factors, replication factors, stabilization factors, antibodies, E6 or E7 from HPV, MUC1, BRCA1, interferons, interleukin (IL-2, IL-4, IL-6, IL-7, IL-12, GM-CSF (Granulocyte Macrophage Colony Stimulating Factor), the tk gene from Herpes Simplex type 1 virus (HSV-1), p53 or VEGF. The polynucleotide can also code for an antibody. In this regard, the term "antibody" encompasses whole immunoglobulins of any class, chimeric antibodies and hybrid antibodies with dual or multiple antigen or epitope specificities, and fragments, such as F(ab)'$_2$, Fab', Fab including hybrid fragments and anti-idiotypes (US 4,699,880). Advantageously said DNA encodes all or part of a polypeptide which is an immunity conferring polypeptide and acts as endogenous immunogens to provoke a humoral or cellular response, or both, against infectious agents, including intracellular viruses, and also against tumor cells. An "immunity conferring polypeptide" means that said polypeptide when it is expressed in the transfected cells will participate in an immune response in the treated patient. More specifically, said polypeptide expressed in macropinocyte cells such as APCs will be processed and the resulting fragments will be presented on the surface of these cells by MHC class I and/or II molecules in order to elicit a specific immune response.

According to a preferred embodiment, the anti-polynucleotide antibody and the polynucleotide comprised in the immuno complex are an anti-DNA antibody and DNA, respectively.

In another preferred embodiment, the antibody included in the immuno complex shows a dissociation rate constant ($K_d$) which is at the most equal to $8 \times 10^{-7}$, preferably at the most equal to $10 \times 10^{-7}$.

The dissociation rate constant ($K_d$) values of the antibodies can be calculated by using for example the inhibition assay described in Friquet et al. (J. Immunol. Methods 77 (1985), 305-319). This constant illustrates the repulsive non-covalent forces resulting from the interaction of the antibody reacting site and its corresponding antigenic determinant at equilibrium. When these repulsive forces ($k_a$) are low and the attractive forces ($k_a$) maximized, the resulting affinity ($K = k_a/k_a$) of the antibody-antigen reaction will be high. This constant and its determination for an antibody used in the present invention are well known by those skilled in the art. This skilled person can furthermore determine, without undue experimentation, for such an antibody, characterized by a certain dissociation rate constant, and for an antigen, the ratio of antibody to polynucleotide which allows to generate an immuno complex according to the present invention. According to Example 1, the skilled man can test antibody dilution ranges combined with a fixed quantity of polynucleotide, measure the complex sizes by known methods and finally determine the ratio of said antibody to said polynucleotide for producing immuno complexes of suitable size. This ratio may be adapted according to the nature (complete antibody or fragment of an antibody) or the properties (dissociation rate constant) of the antibody. For example, the weight ratio can be 50:1. A skilled person is capable of handling these minor adjustments. It is also possible to use a mixture of different anti-polynucleotide antibodies, with different dissociation constant, different epitopes specificities,etc.

According to a particularly preferred embodiment, the immuno complex further comprises a targeting element which can mediate attachment of said immuno complex to the surface of the target cell. This targeting element may be a part of the antibody or of the polynucleotide of said immuno complex. For example, this targeting element may be capable of mediating attachment of the immuno complex to a receptor selected from the group consisting of Fc , FcRI, FcRII, FcRIII, complement receptors, immunoglogulin A or E receptors and macrophage mannose receptor. This attachment to the surface of the cell, although not necessary, may facilitate the macropinocytosis event since the immuno complex would be located in close proximity to the target cell.

In a preferred embodiment of the use of the present invention the macropinocyte target cell is a macrophage or a dentritic cell.

In a further preferred embodiment the composition prepared according to the use of the invention can be used in a method for the therapeutic treatment of humans or animals, preferably in a vaccination method. In this particular case, the composition may also comprise a pharmaceutically acceptable injectable carrier (for examples, see Remington's Pharmaceutical sciences, 16$^{th}$ ed. (1980), Mack Publishing Co). The carrier is preferably isotonic, hypotonic or weakly hypertonic and has a relatively low ionic strength, such as provided by a sucrose solution. Furthermore, it may contain any relevant solvents, aqueous or partly aqueous liquid carriers comprising sterile, pyrogen-free water, dispersion media, coatings, and equivalents, or diluents (e.g;, Tris-HCl, acetate, phosphate), emulsifiers, solubilizers or adjuvants. The pH of the pharmaceutical preparation is suitably adjusted and buffered in order to be useful in in vivo applications.

In a preferred embodiment the composition prepared in accordance with the use of the present invention is in a form for administration into a vertebrate tissue. These tissues include those of muscle, skin, nose, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor etc. Cells where the transfection of a foreign polynucleotide would be obtained are those found in each of the listed target tissues ( hematopoïetic cells, etc.). The administration may, for example, be made by subdermal, intravenous, intramuscular, intracerebral, intratracheal, intraarterial, intraperitoneal, intravesical, intrapleural, intracoronary or intratumoral injection, preferably intradermal or intranasal, with a syringe or other devices.

In another aspect the present invention also relates to a process for introducing a polynucleotide into a macropinocyte target cell wherein said process comprises contacting said cell with at least one composition prepared according to the use of the present invention. This process may be applied by direct administration of said composition to the cell *in vivo*, or by *in vitro* treatment of the cell which may have been extracted from the subject to be treated and by then re-introducing it into the subject (*ex vivo* process). This process can also be implemented on cultured cells (*in vitro*).

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the claims, the invention may be practiced otherwise than as specifically described.

The following example illustrates the invention.

## Example 1

### Preparation of plasmid/antibody complexes

The plasmid/antibody complexes are obtained by adding appropriate amounts of antibody to the plasmid in water or in Tris-buffered saline.
The plasmid comprises a reporter gene such as a gene coding for, e.g., luciferase or beta-galactosidase. The plasmid pTG11033 (European patent application 98 11 2151.0) encoding the *Photinus pyralis* luciferase gene under the control of the cytomegalovirus enhancer/promoter and containing the intron HMG1 is preferably used. Many anti-DNA antibodies are commercially available. The following can be purchased from Interchim: purified mice IgG2b anti-DNA (intercolated) (reference: H55240M), purified mice IgG1,k anti-DNA (intercolated (reference: H55402M), purified mice IgG2b,k anti-DNA (single and double stranded) (reference: H55124M), purified mice IgG2a anti-DNA (single and double stranded) (reference: M11025M), purified sheep anti-DNA (Z) (reference: M20130 E or S). Plasmid preparations at various concentrations are used: 10 µg/ml, 1 µg/ml, 0.10 µg/ml and 0.010 µg/ml. These plasmid preparations are incubated with various amounts of antibody in order to obtain an antibody concentration in the preparation of: 40 µg/µg DNA, 4 µg/µg DNA, 0.40 µg/µg DNA 0.040 µg/µg DNA, in PBS for 15 minutes at room temperature.
The particle size of the formed complexes is determined by dynamic laser light scattering. The concentration of the DNA or preferably of the added antibody, can then be adjusted in order to obtain a suitable complex particle size (0.5 - 6 µm).
The reaction mixtures are then added to wells of 24 well plates that comprise mouse dendritic cells cultured according to published methods in conventional culture medium.
After 48 h the medium is removed, 100 µl lysis buffer (Promega) are added and cells are frozen at -80°C until analysis for luciferase activity. 20 µl of the supernatant are analyzed using the luciferase assay system (Promega) in 96 multi-well plates (Biolumat LB 9500, Berthold, Wilbach, Germany). Values are given as mean relative light units (RLU) per mg of cell protein (BCA assay, Pierce).

## Claims

1. Use of an immuno complex, which comprises at least one antibody or a reactive part thereof and at least one polynucleotide, for the preparation of a therapeutic composition for the introduction of a polynucleotide into a macropinocyte target cell wherein said immuno complex has a particle size selected between 0.5 µm and 6 µm.

2. The use of claim 1, wherein said immuno complex has a particle size of at least 1 µm.

3. The use of claim 1 or 2, wherein said antibody is an anti-polynucleotide antibody.

4. The use of claim 3, wherein said anti-polynucleotide antibody is a monoreactive antibody.

5. The use of claim 3, wherein said anti-polynucleotide antibody is a polyreactive antibody.

6. The use of claim 5, wherein said anti-polynucleotide antibody recognizes a polynucleotide molecule and at least one antigenic compound formulated with said polynucleotide.

7. The use of claim 1 or 2, wherein said antibody is an antibody which does not recognize said polynucleotide but which recognizes at least one of antigenic compound formulated with the polynucleotide.

8. The use of any one of claims 1 to 7, wherein said antibody is a monoclonal antibody.

9. The use of any one of claims 1 to 7, wherein said antibody is an anti-DNA antibody and is isolated from a mammal with an autoimmuno disease.

10. The use of any one of claims 1 to 9, wherein said antibody is an immunoglobuline G (IgG).

11. The use of any one of claims 1 to 10, wherein said reactive part of the antibody is selected from the group consisting of $F(ab')_2$, $F(ab)_2$, Fab', Fab, Fv, sFv and a minimal recognition unit.

12. The use of any one of claims 1 to 11, wherein said polynucleotide is naked polynucleotide.

13. The use of any one of claims 1 to 11, wherein said polynucleotide is formulated with at least one antigenic compound.

14. The use of claim 13, wherein said antigenic compound is selected from the group consisting of polypeptides, cationic lipids and cationic polymers.

15. The use of any one of claims 1 to 14, wherein said polynucleotide contains a gene encoding an immunity conferring polypeptide.

16. The use of any one of claims 1 to 15, wherein said anti-polynucleotide antibody is an anti-DNA antibody and said polynucleotide is DNA.

17. The use of any one of claims 1 to 16, wherein said antibody shows a dissociation rate constant ($K_d$) which is at the most equal to $8 \times 10^{-7}$.

18. The use of any one of claims 1 to 16, wherein said antibody shows a dissociation rate constant ($K_d$) which is at the most equal to $10 \times 10^{-7}$.

19. The use of any one of claims 1 to 18, wherein said immuno complex further comprises a targeting element which can mediate attachment of said immuno complex to the surface of the target cell.

20. The use of claim 19, wherein said targeting element is a part of the antibody of said immuno complex.

21. The use of claim 19, wherein said targeting element is a part of the polynucleotide of said immuno complex.

22. The use of any one of claims 19 to 21, wherein said targeting element can mediate attachment of the immuno complex to a receptor selected from the group consisting of Fc , FcRI, FcRII, FcRIII, complement receptors, immunoglogulin A or E receptors and macrophage mannose receptor.

23. The use of any one of claims 1 to 22, wherein said macropinocyte target cell is selected from the group consisting of macrophages and dendritic cells.

24. The use of any one of claims 1 to 23, wherein said composition further comprises a pharmaceutically acceptable injectable carrier.

25. The use of any one of claims 1 to 24, wherein said composition is implemented in a method for the vaccination of the human or animal body.

26. An in vitro process for introducing a polynucleotide into a macropinocyte target cell wherein said process comprises contacting said cells with at least one composition as defined in any one of claims 1 to 25.

**European Patent Office** **EUROPEAN SEARCH REPORT**

Application Number

EP 98 40 3057

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y,D | WO 97 02840 A (BUTTIN GERARD ;NATO FARIDABANO (FR); AVRAMEAS STRATIS (FR); PASTEU) 30 January 1997<br>* the whole document * | 1-26 | C12N15/87<br>A61K48/00 |
| Y,D | A. AVRAMEAS ET AL.: "Polyreactive anti-DNA monoclonal antibodies and a derived peptide as vectors for the intracytoplasmic and intranuclear translocation of macromolecules."<br>PROC. NATL. ACAD. SCI. USA,<br>vol. 95, May 1998, pages 5601-5606,<br>XP002099626<br>* the whole document * | 1-26 | |
| Y | KOVAL, MICHAEL (1) ET AL: "Size of IgG-opsonized particles determines macrophage response during internalization."<br>EXPERIMENTAL CELL RESEARCH, (JULY 10, 1998) VOL. 242, NO. 1, PP. 265-273. ISSN: 0014-4827., XP002099627<br>* the whole document * | 1-26 | |
| Y | LARSSON, M. (1) ET AL: "Human dendritic cells handling of binding, uptake and degradation of free and IgG-immune complexed dinitrophenylated human serum albumin in vitro."<br>IMMUNOLOGY, (1997) VOL. 90, NO. 1, PP. 138-146. ISSN: 0019-2805., XP002099628<br>* the whole document * | 1-26 | |

TECHNICAL FIELDS SEARCHED (Int.Cl.6)

C12N

-/--

The present search report has been drawn up for all claims

| Place of search | Date of completion of the search | Examiner |
|---|---|---|
| THE HAGUE | 3 May 1999 | Hix, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

# EUROPEAN SEARCH REPORT

**Application Number**

EP 98 40 3057

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | GAO, XIANG ET AL: "Potentiation of Cationic Liposome-Mediated Gene Delivery by Polycations" BIOCHEMISTRY (1996), 35(3), 1027-36 CODEN: BICHAW;ISSN: 0006-2960,1996, XP002099629 * the whole document * | 1-26 | |
| Y | E. WAGNER ET AL.: "Transferrin-polycation-DNA complexes: The effect of polycations on the structure of the complex and DNA delivery to cells." PROC. NATL. ACAD. SCI. USA., vol. 88, May 1991, pages 4255-4259, XP002099630 * the whole document * | 1-26 | |
| Y | AKHTAR S. ET AL: "Antisense oligonucleotide delivery to cultured macrophages is improved by incorporation into sustained-release biodegradable polymer microspheres." INTERNATIONAL JOURNAL OF PHARMACEUTICS, (1997) 151/1 (57-67). REFS: 49 ISSN: 0378-5173 CODEN: IJPHDE, XP002099631 Netherlands * the whole document * | 1-26 | |
| A | LUTZ M B ET AL: "Intracellular routes and selective retention of antigens in mildly acidic cathepsin D/lysosome-associated membrane protein-1/MHC class II-positive vesicles in immature dendritic cells." JOURNAL OF IMMUNOLOGY, (1997 OCT 15) 159 (8) 3707-16. JOURNAL CODE: IFB. ISSN: 0022-1767., XP002099632 United States * the whole document * | | TECHNICAL FIELDS SEARCHED (Int.Cl.6) |

-/--

The present search report has been drawn up for all claims

| Place of search | Date of completion of the search | Examiner |
|---|---|---|
| THE HAGUE | 3 May 1999 | Hix, R |

EPO FORM 1503 03 82 (P04C01)

**European Patent Office**

**EUROPEAN SEARCH REPORT**

Application Number

EP 98 40 3057

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | HOLEVINSKY K O ET AL: "Membrane capacitance changes associated with particle uptake during phagocytosis in macrophages." BIOPHYSICAL JOURNAL, (1998 NOV) 75 (5) 2577-86. JOURNAL CODE: A5S. ISSN: 0006-3495., XP002099633 United States * the whole document * | | |
| A,D | J.C. PERALES ET AL.: "An evaluation of receptor-mediated gene transfer using synthetic DNA-ligand complexes." EUR. J. BIOCHEM., vol. 226, no. 2, December 1994, pages 255-266, XP002099634 * the whole document * | | |
| E,D | EP 0 890 362 A (TRANSGENE SA) 13 January 1999 * the whole document * | 1-26 | |
| A,D | C. WASHINGTON: "Particle size analysis in Pharmeceuticals and other industries." ELLIS HORWOOD SERIES IN PHARMACEUTICAL TECHNOLOGY,1992, pages 135-169, XP002100862 New York * the whole document * | | TECHNICAL FIELDS SEARCHED (Int.Cl.6) |

The present search report has been drawn up for all claims

| Place of search | Date of completion of the search | Examiner |
|---|---|---|
| THE HAGUE | 3 May 1999 | Hix, R |

EPO FORM 1503 03.82 (P04C01)

## ANNEX TO THE EUROPEAN SEARCH REPORT
## ON EUROPEAN PATENT APPLICATION NO.

EP 98 40 3057

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-1999

| Patent document cited in search report | Publication date | Patent family member(s) | Publication date |
|---|---|---|---|
| WO 9702840 A | 30-01-1997 | FR 2736642 A<br>CA 2224413 A<br>EP 0837699 A | 17-01-1997<br>30-01-1997<br>29-04-1998 |
| EP 0890362 A | 13-01-1999 | AU 7394298 A | 28-01-1999 |

EPO FORM P0459

For more details about this annex : see Official Journal of the European Patent Office, No. 12/82